# EUROPÄISCHE PATENTSCHRIFT

(11) **EP 0 914 512 B1**
(45) Veröffentlichungstag und Bekanntmachung des Hinweises auf die Patenterteilung: **17.09.2003**
(21) Anmeldenummer: 97931815.1
(22) Anmeldetag: 15.07.1997
(51) Int. Cl.: D06M 13/148, D06M 13/224, D06M 15/03

(54) **VERFAHREN ZUR HYDROPHILEN AUSRÜSTUNG VON FASERN ODER VLIESSTOFFEN**
PROCESS FOR PROVIDING FIBRES OR NONWOVENS WITH A HYDROPHILIC COATING
PROCEDE POUR MUNIR DES FIBRES OU DES NON TISSES D'UN REVETEMENT HYDROPHILE

(30) Priorität: 23.07.1996 DE 19629667
(43) Veröffentlichungstag der Anmeldung: 12.05.1999
(73) Patentinhaber: Cognis Deutschland GmbH & Co. KG, 40589 Düsseldorf (DE)
(72) Erfinder: MATHIS, Raymond, D-40627 Düsseldorf (DE)
(86) Internationale Anmeldenummer: EP9703783
(87) Internationale Veröffentlichungsnummer: WO98003716

(56) Entgegenhaltungen:
- EP-A- 0 372 890
- EP-A- 0 395 099
- DE-A- 3 702 286
- DE-C- 3 309 530
- US-A- 5 045 387
- DISCLOSED ANONYMOUSLY: "DURABLE HYDROPHILIC FINISHES FOR OLEFINIC NONWOVENS, FILMS (INCLUDING APERTURED) AND LAMINATES IN DISPOSABLE ARTICLES" RESEARCH DISCLOSURE, Nr. 353, 1.September 1993, EMSWORTH, GB, Seite 593 XP000402940

## Beschreibung

Die vorliegende Anmeldung betrifft ein Verfahren zur hydrophilen Ausrüstung von Fasern, die ausschließlich oder überwiegend Polyolefine oder Polyester enthalten oder Vliesstoffe, die überwiegend solche Fasern enthalten sowie Fasern oder Vliesstoffe, die nach diesem Verfahren hydrophil ausgerüstet werden.

Bei der Herstellung von Hygieneartikeln, wie Windeln oder Damenbinden, werden absorbierende Materialien verwendet, um wäßrige Flüssigkeiten aufzunehmen. Um den direkten Kontakt mit dem absorbierenden Material beim Tragen zu verhindern und den Tragekomfort zu erhöhen, wird dieses Material mit einem dünnen, wasserdurchlässigen Vliesstoff umhüllt. Derartige Vliesstoffe werden üblicherweise aus synthetischen Fasern, wie Polyolefin- oder Polyesterfasern hergestellt, da diese Fasern preiswert zu produzieren sind, gute mechanische Eigenschaften aufweisen und im Fall von Polyolefin thermisch verfestigbar sind. Allerdings eignen sich unbehandelte Polyolefin- oder Polyesterfasern für diesen Einsatzzweck nicht, da sie aufgrund ihrer hydrophoben Oberfläche keine ausreichende Durchlässigkeit für wäßrige Flüssigkeiten aufweisen. Zu diesem Zweck muß die Faseroberfläche durch eine entsprechende Präparation hydrophil ausgerüstet werden. Gewünscht ist weiterhin, daß die hydrophile Ausrüstung der Faser möglichst lange erhalten bleibt, ohne daß die Wasserdurchlässigkeit des Vliesstoffs verringert wird. Werden derartige Vliesstoffe beispielsweise in Windeln verarbeitet, können diese mehrfach beansprucht werden, ohne undicht zu werden. Auf diese Weise wird die Tragezeit der Windeln erhöht und der durch verbrauchte Windeln verursachte Abfall verringert.

Die US 5,045,387 beschreibt beispielsweise ein Mittel zur hydrophilen Ausrüstung von Polyolefinfasem, welches eine Mischung aus einem alkoxyliertem Ricinolsäurederivat, einem hydrierten Ricinolsäurederivat, einer C₁₈-Fettsäure und einem polyalkoxylierten Polymethylsiloxan enthält. Die EP 372 890 B 1 offenbart Fasern, die Polyolefine oder Polyester umfassen und mit einem Mittel, welches ein Fettsäurediethanolamid, ein polyethermodifiziertes Silicon, einen Sorbitan-Fettsäureester und ein Metallsalz eines Alkylsulfonats enthalten, behandelt wurden. Der Nachteil derartiger Präparationen ist vor allem in ihrem hohen Preis zu sehen. Weiterhin können Fasern, die mit derartigen Mittel ausgerüstet werden, ein schlechteres Verhalten bei der Vliesherstellung, insbesondere beim Thermobondieren zeigen, was zu einer verringerten Vliesfestigkeit führt. Aus der EP 395 099 A2 sind absorbierende Materialien, insbesondere Tampons aus Rayon- oder Polyesterfasern bekannt, die mit Glycerinmonolaurat als bakteriostatischer, toxinhemmender Komponente ausgerüstet sind. Eine Hydrophilierung der Fasermaterialien wird nicht beschrieben. Die DE 33 09 530 C1 beschreibt hygienische Absorptionsvorlagen, wie Windeln oder Tampons, die mit einer flüssigkeitsdurchlässigen Abdeckfolie versehen sind, welche mit einer Mischung aus Triglyceriden und/oder Partialglyceriden der Kokosfettsäure mit 8 bis 18 C-Atomen als pflegender Komponente imprägniert ist. Bei Windeln, die eine flüssigkeitsdurchlässige Abdeckfolie nach der Lehre der DE-Schrift aufweisen, erhöht sich aber die Ansaugzeit für eine wässerige Natriumchloridlösung bei 20 °C um 50 %.

In dem Artikel "Durable hydrophilie finishes for olefine non wovens, films and Zaminates in disposable articles" (Reseach Disclosure, Nr.353, 1993, Emsworth S. 593) wird die Ausrüstung synthetischer Fasern mit ethoxglichen Polyolestern beschrieben, wobei die Application in gelöster form erfolgt.

Die Aufgabe der vorliegenden Erfindung bestand darin, ein verbessertes Verfahren bereitzustellen, mit dem Polyolefin- oder Polyester enthaltende Fasern oder Vliesstoffe, die solche Fasern enthalten, hydrophil ausgerüstet werden können, wobei die hydrophile Ausrüstung auch nach mehrmaliger Benetzung erhalten bleiben soll.

Es wurde nun gefunden, daß diese Anforderungen durch ein Verfahren erfüllt werden, bei dem man die Fasern oder Vliesstoffe mit einer Präparation behandelt, die Monoester aus Glycerin und bestimmten Fettsäuren enthält.

Gegenstand der Erfindung ist daher ein Verfahren zur hydrophilen Ausrüstung von Fasern die ausschließlich oder überwiegend Polyolefine oder Polyester enthalten oder Vliesstoffen, die überwiegend solche Fasern enthalten, wobei die Fasern oder Vliesstoffe mit einer wäßrigen Dispersion einer Präparation behandelt werden, die 75 bis 100 Gew.-%, bezogen auf das Gewicht der Präparation, mindestens eines Monoesters aus Glycerin und einer Fettsäure mit 6 bis 14 Kohlenstoffatomen und gegebenenfalls bis zu 25 Gew.-% mindestens eines Alkylglykosids mit der allgemeinen Formel RO(G)ₓ in der R einen primären geradkettigen oder methylverzweigten aliphatischen Rest mit 8 bis 22 C-Atomen bedeutet und G für eine Glykosideinheit mit 5 oder 6 C-Atomen steht und x eine Zahl zwischen 1 und 10 bedeutet, enthält.

Das erfindungsgemäße Verfahren eignet sich für Fasern, die ausschließlich oder überwiegend, das heißt zu mehr als 50 Gew.-%, Polyolefine oder Polyester enthalten und für Vliesstoffe, die überwiegend solche Fasern enthalten, wobei Fasern, die ausschließlich Polyolefine oder Polyester enthalten, bevorzugt sind. Besonders geeignet sind Vliesstoffe, die zu 100 Gew.-% aus Polyolefin- oder Polyesterfasern bestehen. Polyolefinfasem gehören zu den am häufigsten zur Herstellung von Vliesen eingesetzten Fasern. Beispiel für geeignete Polyolefine sind Polypropylen, Polyethylen oder Copolymere aus Ethylen oder Propylen mit Butadien. Weiterhin werden auch Polyesterfasern, hauptsächlich Polyethylenterephthalatfasern, verwendet. Es können neben den genannten Fasertypen auch andere zu Herstellung von Vliesen geeigneten synthetischen Fasern verwendet werden, beispielsweise Fasern aus Nylon®. Insbesondere geeignet sind auch Fasern, die aus zwei oder mehr Komponenten bestehen, beispielsweise Polyester-Copolyesterfasern oder Polypropylen-Polyethylenfasern.

Die im erfindungsgemäßen Verfahren verwendeten Vliesstoffe können nach allen im Stand der Technik bekannten Verfahren der Vliesherstellung, wie sie beispielsweise in Ullmann's Encyclopedia of Industrial Chemistry, Vol. A 17, VCH Weinheim 1994, Seiten 572 - 581, beschrieben werden, hergestellt werden. Bevorzugt sind dabei Vliese, die entweder nach dem sogenannte "dry laid"- oder dem Spinnvlies- oder spunbond-Verfahren hergestellt wurden. Das "dry laid"-Verfahren geht von Stapelfasern aus, die üblicherweise durch Kardieren in Einzelfasem getrennt und anschließend unter Einsatz eines aerodynamischen oder hydrodynamischen Verfahrens zum unverfestigten Vliesstoff zusammengelegt werden. Dieser wird dann beispielsweise durch eine thermische Behandlung zum fertigen Vlies verbunden (das sogenannte "thermobonding"). Dabei werden die synthetischen Fasern entweder soweit erwärmt, daß deren Oberfläche schmilzt und die Einzelfasem an den Kontakstellen miteinander verbunden werden, oder die Fasern werden mit einem Additiv überzogen, welches bei der Wärmebehandlung schmilzt und so die einzelnen Fasern miteinander verbindet. Durch Abkühlung wird die Verbindung fixiert. Neben diesem Verfahren sind natürlich auch alle anderen Verfahren geeignet, die im Stand der Technik zum Verbinden von Vliesstoffen eingesetzt werden.
Die Spinnvliesbildung geht dagegen von einzelnen Filamenten aus, die nach dem Schmelzspinnverfahren aus extrudierten Polymeren gebildet werden, welche unter hohem Druck durch Spinndüsen gedrückt werden. Die aus den Spinndüsen austretenden Filamente werden gebündelt, gestreckt und zu einem Vlies abgelegt, welches üblicherweise durch "thermobonding" verfestigt wird.
Das erfindungsgemäße Verfahren eignet sich insbesondere für Vliesstoffe, die nach dem Spinnvlies-oder dem "dry laid"-Verfahren hergestellt werden.

Die Fasern oder Vliesstoffe werden im erfindungsgemäßen Verfahren mit einer Präparation, die mindestens einen Monoester aus Glycerin und einer C₆₋₁₄-Fettsäure enthält, oder einer Mischung, bestehend aus mindestens einem Monoester aus Glycerin und einer C₆₋₁₄-Fettsäure sowie mindestens einem Alkyglykosid, ausgerüstet. Die Präparation wird nach dem erfindungsgemäßen Verfahren in Form einer wäßrigen Dispersion, die vorzugsweise zwischen 5 und 30 Gew.-% der Präparation, bezogen auf das Gesamtgewicht der Dispersion, enthält, auf den unbehandelten Vliesstoff aufgebracht. Dazu können alle in der Textiltechnik üblichen Methoden und Maschinen, beispielsweise ein Foulard, eingesetzt werden. Der Vliesstoff wird zunächst in einem Bad mit der wäßrigen Dispersion in Verbindung gebracht und der so behandelte Vliesstoff zwischen zwei Rollen geführt, wobei das Wasser durch den Druck der Rollen abgepreßt wird. Das erfindungsgemäße Verfahren wird vorzugsweise so gestaltet, daß die Fasern oder Vliesstoffe eine Auflage der Präparation in Mengen von 0,3 bis 2,0 Gew.-%, bezogen auf das Faser- oder Vliesstoffgewicht, erhalten.

Die im erfindungsgemäßen Verfahren eingesetzte Präparation enthält mindestens einen Monoester aus Glycerin und einer C₆₋₁₄-Fettsäure. Der Anteil des Monoglycerids liegt bei mindestens 75 Gew.-%, bezogen auf das Gesamtgewicht der Präparation. Die Fettsäureglyceride sollten dabei von möglichst hoher Reinheit sein, das heißt der Anteil an Di- oder Triestern aus Glycerin und Fettsäure sollte möglichst niedrig liegen. Es können auch Mischungen verschiedener Monoglyceride verwendet werden. Geeignete Fettsäuren sind beispielsweise die Capron-, Capryl-, Caprin-, Laurin- und die Myristinsäure. Ein bevorzugt verwendeter Ester ist der Monoester aus Glycerin und Laurinsäure, das Glycerinmonolaurat.
Als weitere Komponente kann die im erfindungsgemäßen Verfahren verwendete Präparation Alkylglykoside der allgemeinen Formel RO(G)ₓ in Mengen zwischen 5 und 25 Gew.-%, bezogen auf das Gesamtgewicht der Präparation enthalten, wobei in der Formel R für einen primären geradkettigen oder methylverzweigten, insbesondere in 2-Stellung methylverzweigten aliphatischen Rest mit 8 bis 22, vorzugsweise 12 bis 18 C-Atomen steht und G das Symbol ist, das für eine Glykosideinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.. Besonders bevorzugt ist es, Präparationen zu verwenden, die Alkylglykoside in Mengen zwischen 5 und 20 Gew.-%, bezogen auf das Gesamtgewicht der Präparation, enthalten.

Neben den oben beschriebenen Verbindungen, können noch weitere in der Textiltechnik bekannten Stoffe eingesetzt werden, beispielsweise Antistatika oder Gleitmittel. Diese werden üblicherweise in Mengen bis höchstens 20 Gew.-%, bezogen auf das Gesamtgewicht der Präparation, eingesetzt.

Es kann auch vorteilhaft sein das erfindungsgemäßen Verfahren so zu gestalten, daß der Vliesstoff mit einer Präparation behandelt wird, die zusätzlich zu den bereits beschriebenen Komponenten auch quaternäre Esteraminverbindungen der Formel (I) 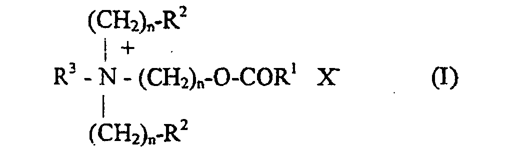 wobei COR¹ für einen aliphatischen Acylrest mit 12 bis 22 Kohlenstoffatomen mit 0, 1, 2 oder 3 Doppelbindungen steht und R² gleich H oder OH sowie R³ ein Alkylrest mit 12 bis 22 Kohlenstoffatomen oder vorzugsweise ein Rest (CH₂)ₙ-O-COR¹ bedeutet, wobei n den Wert 1, 2 oder 3 hat und X entweder ein Halogenid-, Methosulfat- , Methophosphat- oder Phosphation ist, in Mengen zwischen 5 und 25 Gew.-%, bezogen auf die Gesamtmenge der Präparation, enthält.

Nach dem erfindungsgemäßen Verfahren werden hydrophil ausgerüstete Fasern, die ausschließlich oder überwiegend Polyolefine oder Polyester enthalten oder Vliesstoffe, die überwiegend solche Fasern enthalten, hergestellt. Die Fasern oder Vliesstoffe zeigen hydrophile Eigenschaften, die vorzugsweise auch bei mehrmaliger Benetzung erhalten bleiben. Insbesondere weisen die erfindungsgemäß hergestellten Vliesstoffe " liquid strike through"-Zeiten von weniger als 10 Sekunden und insbesondere weniger als 5 Sekunden auf. Unter "liquid strike-through"-Zeiten werden im Rahmen dieser Anmeldung die Zeiten verstanden, die eine bestimmte Menge Wasser oder künstlicher Urin benötigt, um durch das Vlies zu einer absorbierenden Unterschicht zu gelangen. Diese Zeit wird nach der EDANA (europäischen Verband der Vliesstoffhersteller) Testmethode 150.0-84 bestimmt. Für den Einsatz in Windeln oder ähnlichen Hygieneartikeln sollte die "liquid strike through"-Zeit möglichst klein sein, um einen schnellen Transport der Flüssigkeit durch das Vlies zum absorbierenden Material zu gewährleisten. Auf diese Weise bleibt die Vliesoberfläche trocken und führt so zu einem erhöhten Tragekomfort. Die erfindungsgemäß hergestellten Vliesstoffe behalten diese günstige Eigenschaft auch bei mehrmaligem Gebrauch und zeichnen sich weiter dadurch aus, daß sie vorzugsweise bei dreimaliger, insbesondere bei fünfmaliger, hintereinander durchgeführten Bestimmung der "liquid strike through"-Zeit nach der EDANA Testmethode 150.0-8, in jedem Fall eine "liquid strike through"-Zeit von weniger als 10 Sekunden, insbesondere von weniger als 5 Sekunden aufweisen.

### Beispiele

Zur Messung der Hydrophilie der unterschiedlich hergestellten Vliesstoffe wurden die "strike-through˝-Zeiten der Testvliese nach der EDANA Testmethode 150.0-84 gemessen.
Die Messungen wurden mit einem Testgerät mit Zeitaufnehmer (Lister-Tester) der Fa. Lenzing AG durchgeführt. Die Testvliese (Polypropylen-spunbond-Vlies mit einem Gewicht von 20g/m² - Lutrasil® 4420, Fa. Freudenberg) wurden mit der wäßrigen Dispersion der Präparation besprüht. Anschließend wurden die Testvliese bei 70 °C eine Stunde lang getrocknet.
Für die eigentliche Messungen wurden 6 Lagen eines Filterpapiers (Evans-Adlard FF3 WIS 150) unter eine Lage des präparierten Testvliesses gelegt und anschließend mit 5 ml künstlichen Urins (1000 ml dest. H₂O, 9 g NaCl) aus dem Lister-Tester in Kontakt gebracht. Die Messungen wurden bei 20 °C und 65 % relativer Luftfeuchtigkeit durchgeführt. Die Zeit, die die Flüssigkeit braucht, um das Vlies zu durchdringen, wurde am Zeitaufhehmer in Sekunden abgelesen. Um die hydrophilen Eigenschaften der Vliesstoffe bei mehrmaliger Beanspruchung zu messen, wurden das Testvlies nach der ersten Messung, ohne getrocknet zu werden, mit einer neuen Schicht aus Filterpapier unterlegt und die Messung wiederholt. Zeiten von maximal 5 Sekunden bei jeweils 5 Wiederholungen wurden als gut bewertet. Zeiten über 5 Sekunden wurden nicht mehr wiedergegeben.

Die Ergebnisse sind der Tabelle 1 zu entnehmen. Angegeben sind die "strike-through"-Zeiten in Sekunden für Vlies, die mit unterschiedlichen Mitteln präpariert wurden. Wiedergegeben sind Mittelwerte von jeweils 5 Messungen. Die Vliese wurden mit 1.0 und 2,0 Gew.-%, bezogen auf das Vliesstoffgewicht, mit der entsprechenden Präparation belegt.

Getestet wurden Vliesstoffe, die mit den folgenden Mischungen präpariert wurden. Die Angaben in Gew.-% sind bezogen auf das Gesamtgewicht der verwendeten Präparationen.
Die jeweiligen Präparation wurde in Form einer wäßrigen Dispersion verwendet, welche 20 Gew.-% Aktivsubstanz, bezogen auf die wäßrige Dispersion, enthielt.

### Präparation 1:

100 Gew.-% Glycerinmonolaurat

### Präparation 2:

85 Gew.-% Glycerinmonolaurat
15 Gew.-% C8-14 Alkylpolyglucosid

### Präparation 3

80 Gew.-% Glycerinmonolaurat
20 Gew.-% C₈₋₁₄ Alkylpolyglucosid

Zum Vergleich wurde folgende Präparation verwendet:

### Präparation V1:

100 Gew.-% C₈₋₁₄ Alkylpolyglucosid

**Tabelle 1**

| Anzahl der Messungen | | | | | | |
|---|---|---|---|---|---|---|
| | 1 | 2 | 3 | 4 | 5 | 6 |
| Präparation | | | | | | |
| 1 (Auflage 1,0 %) | 3,12 | 3,98 | 4,40* | 5,00* | - | - |
| 1 (Auflage 2,0%) | 3,10 | 3,34 | 3,85 | 4,40 | 4,14 | 3,94 |
| 2 (Auflagel 1,0%) | 2.81 | 3,11 | 4,07 | 4,83* | - | - |
| 2 (Auflage 2,0%) | 3,02 | 3,07 | 3,03 | 3,34 | 4,35 | 3,92 |
| 3 (Auflage 1,0%) | 3,02 | 3,02 | 3,88 | 4,83 | - | - |
| 3 ( Auflage3 2,0 %) | 3,05 | 3,01 | 2,79 | 2,90 | 3,39 | 3,40 |
| V1 (Auflage 1,0 %) | 3,30 | - | - | - | - | - |

| | | | | | | |
|---|---|---|---|---|---|---|
| ^{*} Mittelwert aus vier Messungen | | | | | | |

Man erkennt, daß ein erfindungsgemäß hergestelltes Vlies, das mit der Präparation 1 behandelt wurde, auch bei der vierten Messung sowohl bei einer Auflage von 2,0 % als auch 1,0 % noch "liquid strike through"-Zeiten von höchstens 5 Sekunden zeigt. Dieser Effekt wird durch die Kombination aus Glycerinmonolaurat mit einem Alkylglucosid ebenfalls erzielt, wie der mit den Präparationen 2 und 3 behandelte Vliesstoff zeigt. Die Präparation 3 zeigt dabei die beste Permanenz. Auch ein Vliesstoff, der nur mit einem Alkylpolyglucosid behandelt wurde, wie mit der Präparation V1, zeigt eine gute Hydrophilierung. Diese Ausrüstung bleibt aber bei mehrfacher Belastung nicht erhalten, so daß die "liquid strike through"-Zeiten schon bei der folgenden Messungen deutlich länger wird.

## Patentansprüche

1. Verfahren zur hydrophilen Ausrüstung von Fasern, die ausschließlich oder überwiegend Polyolefine oder Polyester enthalten oder Vliesstoffe, die überwiegend solche Fasern enthalten, **dadurch gekennzeichnet, daß** die Fasern oder Vliesstoffe mit einer wäßrigen Dispersion einer Präparation behandelt werden, die zwischen 75 und 100 Gew.-%, bezogen auf das Gesamtgewicht der Präparation, mindestens eines Monoesters aus Glycerin und einer Fettsäure mit 6 bis 14 Kohlenstoffatomen und gegebenenfalls bis zu 25 Gew.-%, bezogen auf das Gesamtgewicht der Präparation, an Alkylglykosiden der Formel RO(G)ₓ in der R einen primären geradkettigen oder methylverzweigten aliphatischen Rest mit 8 bis 22 C-Atomen bedeutet und G für eine Glykosideinheit mit 5 oder 6 C-Atomen steht und x eine Zahl zwischen 1 und 10 bedeutet, enthält.

2. Verfahren nach Anspruch 1, **dadurch gekennzeichnet, daß** die Präparation Alkylglykoside in Mengen zwischen 5 und 20 Gew.-%, bezogen auf das Gesamtgewicht der Präparation, enthält.

3. Verfahren nach Anspruch 1 oder 2, **dadurch gekennzeichnet, daß** eine wäßrige Dispersion eingesetzt wird, die zwischen 5 und 30 Gew.-% der Präparation, bezogen auf das Gewicht der Dispersion, enthält.

4. Verfahren nach einem der Ansprüche 1 bis 3, **dadurch gekennzeichnet, daß** als Glycerinmonoester Glycerinmonolaurat eingesetzt wird.

## Claims

1. A process for the hydrophilic finishing of fibers, which exclusively or predominantly contain polyolefins or polyesters, or of nonwovens which predominantly contain fibers such as these, **characterized in that** the fibers or nonwovens are treated with an aqueous dispersion of a finish which contains 75 to 100% by weight, based on the total weight of the finish, of at least one monoester of glycerol and a fatty acid containing 6 to 14 carbon atoms and optionally up to 25% by weight, based on the total weight of the finish, of alkyl glycosides corresponding to the formula RO(G)ₓ, in which R is a primary linear or methyl-branched aliphatic radical containing 8 to 22 carbon atoms, G is a glycoside unit containing 5 or 6 carbon atoms and x is a number of 1 to 10.

2. A process as claimed in claim 1, **characterized in that** finish contains alkyl glycosides in quantities of 5 to 20% by weight, based on the total weight of the finish.

3. A process as claimed in claim 1 or 2, **characterized in that** an aqueous dispersion containing between 5 and 30% by weight of the finish, based on the total weight of the dispersion, is used.

4. A process as claimed in any of claims 1 to 3, **characterized in that** glycerol monolaurate is used as the glycerol monoester.

## Revendications

1. Procédé d'apprêtage hydrophile de fibres qui renferment exclusivement ou d'une manière prépondérante des polyoléfines ou des polyesters, ou de non-tissés qui renferment d'une manière prépondérante ces fibres,
**caractérisé en ce que**
les fibres ou les non-tissés sont traités par une dispersion aqueuse d'une préparation qui contient entre 75 et 100 % en poids rapporté au poids total de la préparation, d'au moins un monoester à base de glycérol et d'un acide gras ayant de 6 à 14 atomes de carbone, et éventuellement jusqu'à 25 % en poids rapporté au poids total de la préparation, d'alkylglycosides de formule RO(G)ₓ, dans laquelle R signifie un reste aliphatique primaire à chaîne droite ou ramifiée par un méthyle ayant de 8 à 22 atomes de carbone, et G représente un élément glycosidique ayant 5 ou 6 atomes de carbone, et x signifie un nombre entre 1 et 10.

2. Procédé selon la revendication 1,
**caractérisé en ce que**
la préparation contient des alkylglycosides en quantités comprises entre 5 et 20 % en poids rapporté au poids total de la préparation.

3. Procédé selon l'une quelconque des revendications 1 ou 2,
**caractérisé en ce qu'**
on met en oeuvre une dispersion aqueuse qui renferme entre 5 et 30 % en poids de la préparation rapporté au poids de la dispersion.

4. Procédé selon l'une quelconque des revendications 1 à 3,
**caractérisé en ce que**
comme monoester de glycéryle on met en oeuvre du monolaurate de glycéryle.
